# EUROPEAN PATENT SPECIFICATION

(11) **EP 1 778 319 B1**
(45) Date of publication and mention of the grant of the patent: **08.09.2010**
(21) Application number: 05772305.8
(22) Date of filing: 15.07.2005
(51) Int. Cl.: A61M 25/09

(54) **GUIDEWIRE BEARING MARKINGS SIMPLIFYING CATHETER SELECTION**
FÜHRUNGSDRAHT MIT MARKIERUNGEN ZUR VEREINFACHUNG DER KATHETERAUSWAHL
FIL DE GUIDAGE PORTANT DES MARQUES POUR SIMPLIFIER LA SELECTION DES CATHETERS

(30) Priority: 19.07.2004 US 893847
(43) Date of publication of application: 02.05.2007
(73) Proprietor: Arrow International, Inc., Reading, PA 195605 (US)
(72) Inventor: CASSIDY, Kenneth, Todd, Mockville, NC 27028 (US); MARTEL, Mark, Curran, Belews Creek, NC 27009 (US)
(74) Representative: Minderop, Ralph H.
(86) International application number: PCT/US2005/025297
(87) International publication number: WO 2006/020129

(56) References cited:
- EP-A- 1 203 595
- WO-A-02/28468
- US-A- 5 804 022
- US-A- 6 074 367
- US-A- 6 074 367
- US-A1- 2004 106 878
- US-B1- 6 613 002

## Description

### BACKGROUND OF THE INVENTION

The present invention relates to optimal placement of the distal tip of a catheter during insertion of the catheter into a patient.

For most catheters, there is an ideal location within the patient where the distal tip, i.e. the distal tip or tips, of the catheter should be positioned. Proper placement of the distal tip of the catheter depends directly upon the length of catheter being inserted. If the catheter utilized is too short, the optimal location cannot be reached; if the catheter is too long, the optimal location can be over-shot. Thus, some catheter supplied to doctors include several catheters, each of a different length.

Peripherally Inserted Central Catheters have been utilized by clinicians for several decades. Insertion using the Seldinger technique has been used even longer, primarily for the insertion of subclavian and other chest inserted catheters.

The Seldinger technique begins with obtaining access to a vein with a needle. The needle is hollow and permits a wire to pass through the central bore thereof, after it is determined that the needle has been inserted into the appropriate vein. Specifically, the wire is threaded through a proximal hub of the needle, through the central bore of the needle and into the vein. The wire is often referred to as a "guidewire" since its ultimate purpose is to guide a catheter into place. Once it is determined that the guidewire is in place, the needle may be removed by backing the needle over the guidewire, leaving the guidewire in place. Proper placement of the guidewire may be verified by fluoroscopy.

The guidewire may then be used to allow a dilator to slide into the vein and widen the opening to the skin and vein. The dilator is removed with the guidewire, again, left in place. At this point, a catheter is advanced over the guidewire and the distal end of the catheter is placed in the desired location. The guidewire is then removed.

A catheter introducer may also be used in the Seldinger technique. The introducer is disposed about the dilator device and inserted along with the dilator. When the dilator is removed the introducer remains. The catheter is advanced through the introducer. As the insertion is completed the introducer is pulled out of the skin, around the catheter, and split according to the manufacturer's usage directions.

A frequent problem with many different types of catheters is that, once the catheter is properly placed, the catheter may have a tendency to slip. That is, once placed a catheter will often have a tendency to be pulled out of the patient or be pushed further into the patient. Either situation is, at best, likely to affect the operation of the catheter and, at worst, extraordinarily dangerous for numerous reasons. One technique for firmly anchoring a catheter in place is to create a tunnel under the skin of the patient. The portion of the catheter extending from the incision in the patients body can be routed through this tunnel to greatly decrease the likelihood of the catheter shifting. This subcutaneous tunnel is usually located very close to the incision site and may, in fact, have one end which is precisely the same as the Seldinger incision site.

With any type of Seldinger technique, a critical factor to the ultimate proper placement of the catheter is the length of the catheter. With some types of catheters, it is possible to trim one or both ends of the catheter. In such cases the practitioner may be supplied with a catheter of far greater length than will actually be needed. Once this overly long catheter is inserted into the patient and it is determined that the distal tip of the catheter is properly placed, any extraneous portion of the proximal end of the catheter may be trimmed away.

However, many catheters have specific structural constraints at both ends. As a result of these structural constraints, such catheters may not be trimmed to size. In such cases, several catheters each of a different length should be made available to the practitioner. In addition, a method by which the practitioner can choose from among these catheters of varying lengths is also necessary.

One method for choosing the appropriate catheter length to use in a procedure involves 'marking' and measuring the guidewire. In this method a doctor inserts a guidewire into a patient and determines the proper tip placement using a fluoroscope or other imaging apparatus. Once the tip of the guidewire is determined to be at the desired location, the doctor marks an external portion of the guidewire in some manner, e.g. by 'kinking' the guidewire where it exits the incision in the patient. The guidewire may then be removed and measured from the distal tip to the 'kink' or other mark made by the doctor. The measurement may be accomplished with a standard measuring tape, ruler or the like. In addition, the guidewire may be marked along its length with units of length. However the length is determined, this measurement is used by the doctor to calculate the stock length of catheter to be used in the procedure.

U.S. Patent No. 6,074,367 to Hubbell discloses a permutation on the above marking and measuring technique. The catheter insertion kit disclosed by Hubbell includes a guidewire of predetermined length which has a plurality of markings at predefined intervals. The guidewire is inserted into a body vessel until the tip of the wire is positioned at the desired catheter tip location. The number of markings on the portion of the guidewire remaining outside the body are then counted or otherwise measured and used to calculate the length of wire either outside or inside the body. This length, along with other known factors such as the guidewire length, is then used to choose a stock length of catheter.

### SUMMARY OF THE INVENTION

The purpose of the present invention is to simplify the process for choosing a stock length of catheter to be inserted into a patient.

In one aspect of the present invention, many of the steps of marking and measuring a guidewire and calculations utilizing the measurements are eliminated. In place of the detailed and error prone set of marking, measuring and calculating steps, a set of unique identifiers are printed or otherwise directly and immovable associated with the guidewire. A practitioner determines the stock length of catheter needed in a procedure merely by noting the placement of one unique identifier relative to a standard landmark.

In a preferred embodiment of the present invention the unique identifiers are color coded. In addition, the landmark relative to the unique identifier is the insertion site. That is, the unique identifier which can be seen as closest to the incision from which the guidewire extends from the patient, is used in determining the stock catheter length to be used.

In another preferred embodiment of the present invention, the unique identifier associated with the guidewire may be cross referenced to the stock catheter lengths using a conversion chart. Each unique identifier corresponds to a stock catheter length, this association being determined by referencing a conversion chart.

In a most preferred embodiment of the present invention, the unique identifier associated with the guidewire is also directly associated with the appropriate stock catheter.

In an alternate embodiment of the present invention, the techniques and structures above can be used in combination with not only the insertion, i.e. Seldinger incision, site but also with a subcutaneous tunnel. The guidewire can actually extend through a subcutaneous anchoring tunnel or could be laid on the skin to estimate the placement of the tunnel.

### BRIEF DESCRIPTION OF THE DRAWING

The above and related objects, features and advantages of the present invention will be more fully understood by reference to the following detailed description of the presently preferred, albeit illustrative, embodiments of the present invention when taken in conjunction with the accompanying drawing wherein:

FIG. 1 is an illustration of the guidewire;

FIG. 2 is an illustration of the guidewire in use; and

FIG. 3 is an illustration of the guidewire in use in an alternate embodiment where a subcutaneous anchoring tunnel is also provided.

### DETAILED DESCRIPTION OF THE PREFERRED EMBODIMENTS

A structure and method for using a guidewire to choose the appropriate length of a catheter from a set of stock catheter lengths is described. A broad range of catheter types can be utilized in combination with the guidewire described herein. Chronic hemodialysis catheters are expected to be commonly utilized with regard to the present guidewire structure and method, but central ports, tunneled central catheters, or other catheters requiring a controlled choice of length could also utilize the guidewire and associated method described herein. In addition, the guidewire described herein may be utilized in combination with different sub-structures of this broad range of catheters as well as in combination with a range of catheter insertion methods. Such structures and methods include various tunneling and reverse-tunneling catheters and other methods of securing, i.e. anchoring, a catheter in place.

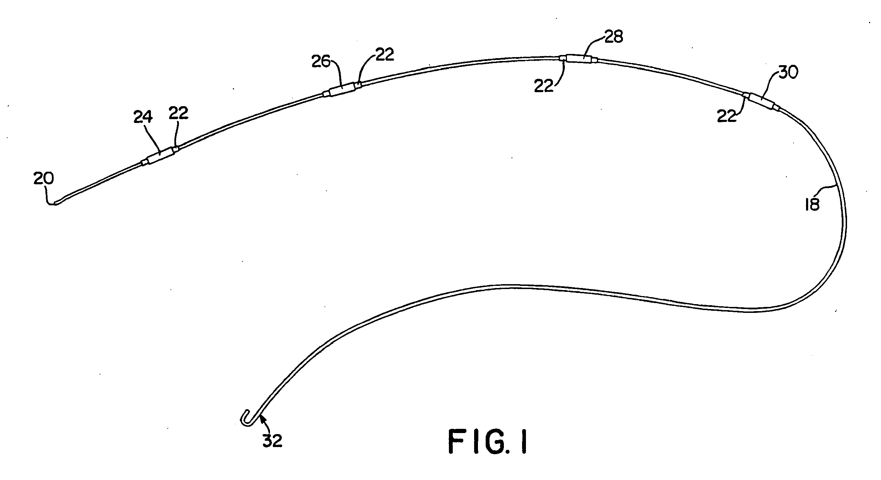

Referring now to the drawings, and in particular to Fig. 1 thereof, therein illustrated is a guidewire 18. The guidewire 18 is of a predetermined length and has a distal end 32 and a proximal end 20. The distal end 32 of the guidewire 18 may be provided with a flexible "J" shape to aid in insertion and reduce trauma to the vasculature of the patient. Alternatively, the distal end may simply be provided with another atraumatic structure, e.g. a simple rounded tip or a soft polymeric tip. The entire catheter and the "J" shaped distal end 32, if provided, are of a sufficiently flexible nature that the circuitous nature of a patient's vasculature will not prevent the wire from being inserted to the desired location.

Guidewire 18 also comprises a plurality of unique identifiers 24, 26, 28, 30. As the name implies, each unique identifier 24, 26, 28, 30 can be easily and quickly distinguished from each and every other unique identifier. By way of example, each of the unique identifiers 24, 26, 28, 30 may be of a different color. The only limitations with regard to which colors may be chosen are that they contrast sufficiently with the color of guidewire 18 and that they be sufficiently different from the other unique identifier colors that easy identification and differentiation is possible. As another example, each unique identifier could be an easily recognized symbol.

In order to provide the greatest possible contrast between each unique identifier 24, 26, 28, 30 and the background color of the guidewire 18 on which the unique identifiers are disposed, a base 22 may also be provided. It is not necessary that each base 22 be unique. By way of example, assuming that the guidewire is some color other than white, each base 22 can be white and each unique identifier disposed over the white base 22. Additionally, a unique symbol may be printed directly onto base 22, thus forming unique identifiers 24, 26, 28, 30.

The base 22 and the unique identifiers 24, 26, 28, 30 can take any one of a number of forms. They may be printed on the guidewire using biocompatible ink or they may be painted on the guidewire using biocompatible paint. Polymeric materials of various types may also be applied to the guidewire to form the base 22 and unique identifiers 24, 26, 28, 30.

One important aspect of the base 22 and unique identifiers 24, 26, 28, 30 is that they present as low a profile as possible. That is, the difference between the diameter of the guidewire and the diameter of the guidewire with the base 22/unique identifiers 24, 26; 28, 30 disposed thereon, should be as small as possible. This is because these structures on the guidewire must sometimes be inserted into or through certain structures, e.g. a needle or incision, along with the guidewire. Although the figures disclose the base 22 and unique identifiers 24, 26, 28, 30 as having a slightly larger diameter than guidewire 18, even this slight difference is merely for the purpose of illustration. In practice, the diameter difference will be so small as to be practically unnoticeable.

Although four unique identifiers are discussed herein, and the examples of colors and symbols are presented as representing these unique identifiers, it should be clearly understood that these embodiments are presented purely by way of example. The number of unique identifiers that are to be disposed on the guidewire is determined by the number of stock catheter lengths from which the catheter of optimal length is being chosen. The specific form which the unique identifier takes is dictated only by the ease with which the identifier can be seen and easily identified by the practitioner during use.

For most catheters it is necessary that the length of the catheter be adjustable. The catheter must be long enough so that the distal end thereof can be placed at a specific point in the patient's anatomy. However, the catheter must be short enough that, when the distal tip is properly placed, the proximal portion is not so long that it could interfere with the patients movement or increase the likelihood of damaging the catheter. Movement or shifting of the catheter, which greatly increases the possibility of infection, can also be decreased by assuring that the catheter portion extending proximally from the incision in the patient is not overly long. Because it is not possible to alter the size of many types of catheters, i.e. by trimming a portion thereof, it is the usual practice to supply catheters in a plurality of lengths. Thus, based on the specific anatomy of the patient and precisely where the medical professional desires the distal tip of the catheter to be placed, a range of sizes are available so that the catheter can be 'fit' to the patient, being mindful of the sizing parameters outlined above. The various sizes made available to the medical professional are referred to herein as "stock sizes", i.e. the sizes that the hospital would have in stock or would be included in a kit supplied by a manufacturer.

The ultimate purpose of the plurality of unique identifiers is to provide the practitioner, i.e. the doctor or other medical professional inserting a catheter into a patient, with a simple and foolproof way of determining the length of catheter that would 'fit' the patient the best. The practitioner would then be able to choose the stock size to be inserted into the patient.

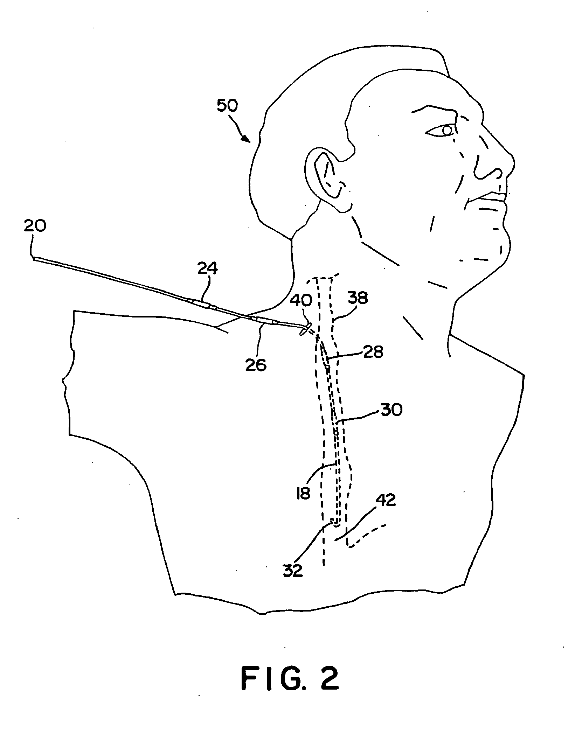

FIG. 2 shows a patient 50 undergoing a catheter insertion. A portion of the patient's vasculature 38 transcutaneous to the patient's neck has been accessed, e.g. utilizing the Seldinger technique, through incision 40. Guidewire 18 has been inserted through incision 40 and into the vasculature of the patient and can be advanced or retracted by the practitioner as desired. It is possible, through well known techniques such a fluoroscopy, for the practitioner to visualize the guidewire 18 and its relationship to many anatomical structures inside the patient.

Point 42 in FIG. 2 is an imaginary point in the vasculature of the patient where the practitioner desires to place the distal tip of a catheter. The practitioner places the distal end 32 of guidewire 18 adjacent point 42, confirming the placement by fluoroscopy or the like. When the distal end 32 of the guidewire 18 is properly placed, at least one of the unique identifiers 22, 24, 26, 28 will remain outside of the patient's body. Some of the unique identifiers may enter the patient through incision 40.

In the specific example of FIG. 2, the practitioner has placed the distal end 32 of guidewire 18 at the desired location 42. As a result of this placement, two of the unique identifiers 28 and 30 are now disposed inside the patient and cannot be seen by the practitioner. The two other unique identifiers 24 and 26 are located outside of the patient and can be seen by the practitioner. Based on the placement of the guidewire 18, the practitioner may now choose the optimally sized, i.e. not too long and not too short, stock length of catheter to be inserted into the patient (i.e. located outside the patient's body). This choice will be dictated by whichever unique identifier is located closest to incision 40 while still being visible to the practitioner. Again using the example of FIG. 2, unique identifier 26 is located closest to incision 40 and can still be seen by the practitioner since it is outside the body. Thus, the stock catheter length that corresponds to unique identifier 26 is retrieved and is inserted into the patient. This insertion may be accomplished using guidewire 18, i.e. the chosen catheter is disposed over the guidewire 18. Such a procedure is well known to practitioners.

There are many possible ways of corresponding a unique identifier to a stock catheter length. A chart could be provided to the practitioner that lists the unique identifier and the stock number of the catheter of the appropriate size that corresponds to that unique identifier. Once the guidewire is placed and the proper unique identifier is noted by the practitioner, the chart may be referenced to determine which stock catheter length should be utilized.

The unique identifiers and chart may be color coded to further ease the cross referencing. Each unique identifier would be a different easily distinguishable color and the accompanying chart would be color coded to identify the appropriate stock catheter.

Direct correlation of the unique identifier to the collection of stock catheters may also by utilized. The unique identifiers, e.g. collection of colors or symbols, marked on the guidewire 18 at locations 24, 26, 28, 30 may also be directly marked on the packaging of the catheters corresponding to the unique identifiers. For example, if unique identifier 26 happened to be the color orange then the label on the packaging of the catheter which corresponds to that unique identifier can either be partly or wholly orange in color. Alternatively, the label on the shelf where the corresponding catheter is stored may be coded with the color orange.

Another embodiment of the present guidewire 18 involves directly printing the size of the appropriate catheter on the neutral base 22. The unique identifier would thus be a number of centimeters corresponding to the various stock lengths provided. If the catheters are provided in lengths of 21 cm, 25 cm, 29 cm and 33 cm, then the unique identifiers printed on base 22 would be the numbers "21", "25", "29" and "33", with or without their corresponding unit values.

It is almost always necessary to assure that a catheter is anchored in place, i.e. is prevented from being pulled out of a patient or being forced too far into the patient. This anchoring or securing of a catheter in place is often accomplished through the use of a subcutaneous tunnel. This subcutaneous tunnel has a first end thereof at or near the incision site where the catheter extends from the patient's body and a second end a certain distance away from the first end. A portion of the catheter is disposed through this subcutaneous tunnel and provides frictional anchoring of the catheter in place. This anchoring is often increased by the use of structures on the external surface of the catheter which allow subcutaneous tissue to 'attach' to the catheter.

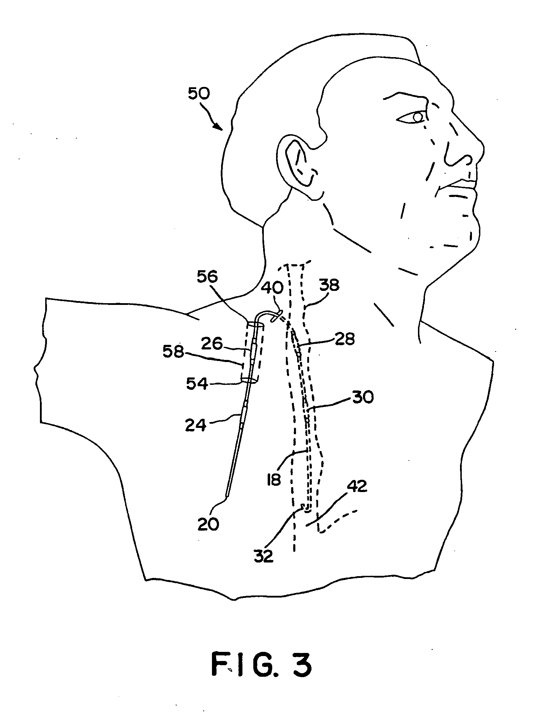

The utilization of a subcutaneous tunnel in anchoring a catheter will, of course, have an impact on the length of catheter needed for a particular patient. FIG. 3 shows guidewire 18 of the same structure as has been previously described. In addition, the internal anatomy and incision 40 of the patient are also identical to what has been previously described. The difference in FIG. 3 is that a subcutaneous tunnel 58 has been formed under the skin of the patient 50 for the purpose of securing or anchoring the catheter in place. Tunnel 58 has two ends 54 and 56. The end 56 of tunnel 58 closest to the incision site 40 may be coextensive, i.e. the same as, the incision site 40. In such a case the first end of the tunnel 56 is the incision site 40 and the second end of the tunnel 54 remains a certain distance therefrom.

Obviously, the use of a subcutaneous tunnel will alter the required length of catheter to be used. In the specific example of FIG. 3, the practitioner has placed the distal end 32 of guidewire 18 at the desired location 42. As a result of this placement, two of the unique identifiers 28 and 30 are now disposed inside the vasculature of the patient and cannot be seen by the practitioner. A third unique identifier 26 is located inside the subcutaneous tunnel and also cannot be seen by the practitioner. Unique identifier 24 is located outside of the patient and can be seen by the practitioner. Based on the placement of the guidewire 18, the practitioner may now choose the optimally sized, i.e. not too long and not too short, stock length of catheter to be inserted into the patient (i.e. located outside the patient's body). This choice will be dictated by whichever unique identifier is located closest to second end 54 of subcutaneous tunnel 58 while still being visible to the practitioner. Again using the example of FIG. 3, unique identifier 24 is located closest to second end 54 of subcutaneous tunnel 58 and can still be seen by the practitioner. Thus, the stock catheter length that corresponds to unique identifier 24 is retrieved and is inserted into the patient. This insertion may be accomplished using guidewire 18, i.e. the chosen catheter is disposed over the guidewire 18. Such a procedure is well known to practitioners.

It is not necessary for the guidewire 18 to actually be disposed through subcutaneous tunnel 58 for the choice of catheter to be made. It is possible for the practitioner to merely lay the guidewire 18 on the skin surface of the patient 50 and calculate where the second end 54 of subcutaneous tunnel 58 will be. The practitioner can be instructed to assume that the portion of guidewire 18 which overlies the proposed or actual subcutaneous tunnel 58 is 'covered' by the skin and to use the first unique identifier that the practitioner knows is not covered by the skin in choosing the appropriate stock catheter length.

Another embodiment of the present guidewire 18 involves its inclusion in a kit of other materials described herein or which would be obvious to a practitioner as necessary for inserting a catheter utilizing guidewire 18. This kit may include the components necessary for performing the insertion of a catheter utilizing the Seldinger technique, including the needle, dilator(s) and sheath introducer, among other components. It is also possible to supply several stock sizes of catheter in such a kit. Each of these catheters would, in conforming to the foregoing, correspond to a unique identifier on the enclosed guidewire. Thus, each kit could include all of the necessary components for the insertion of a catheter. A kit including the selection of guidewires would be particularly attractive in situations where a selection of separately packaged stock guidewires is not available.

Now that the present invention has been described in detail, various modifications and improvements thereon will become readily apparent to those skilled in the art. Accordingly, the scope of the present invention is to be construed broadly and limited only by the appended claims, and not by the foregoing specification.

## Claims

1. A catheter introducer system comprising: a flexible catheter guidewire (18) having a distal end (32), a proximal end (20) and a central portion of a predetermined length having disposed thereon a plurality of unique identifiers (24, 26, 28, 30), each unique identifier (24, 26, 28, 30) being easily distinguishable from each and every other unique identifier (24, 26, 28, 30) on the catheter guidewire (18), a plurality of catheters of varying stock lengths, each stock catheter length being associated with a catheter identifier that has a direct visual correspondence with one unique identifier (24, 26, 28, 30) on the guidewire (18).

2. The catheter introducer system according to claim 1 wherein: each of the plurality of unique identifiers (24, 26, 28, 30) is of a color.

3. The catheter introducer system according to claim 1 wherein: each of the plurality of unique identifiers (24, 26, 28, 30) is of a symbol.

4. The catheter introducer system according to claim 1 wherein: each of the plurality of unique identifiers (24, 26, 28, 30) is of a code, the code comprising one or more symbols.

5. The catheter introducer system according to claim 1 wherein: each of the plurality of unique identifiers (24, 26, 28, 30) is printed on a base (22).

6. The catheter introducer system according to claim 5 wherein: the base (22) is of such a color that it provides a contrast between a color of the guidewire (18) and a color of the unique identifier (24, 26, 28, 30).

7. The catheter introducer system according to claim 5 wherein: the base (22) is white.

8. The catheter introducer system according to claim 1 wherein: the distal end (32) of the guidewire (18) is provided with a flexible J shape.

9. The catheter introducer system according to claim 1 wherein: the catheter is associated with the guidewire unique identifier (24, 26, 28, 30) to which it corresponds through a catheter unique identifier printed directly on the catheter.

10. The catheter introducer system according to claim 1 wherein: each catheter is contained in a catheter package and is associated with the guidewire unique identifier (24, 26, 28, 30) to which it corresponds through a catheter unique identifier printed the catheter package.

## Patentansprüche

1. Kathetereinführsystem, enthaltend: einen flexiblen Katheterführungsdraht (18), der ein distales Ende (32), ein proximales Ende (20) und einen Mittelabschnitt mit einer vorbestimmten Länge hat, auf dem eine Vielzahl von einzigartigen Kennzeichen (24, 26, 28,30) angeordnet sind, wobei jedes einzigartige Kennzeichen (24, 26, 28, 30) von jedem der jeweiligen anderen Kennzeichen (24, 26, 28, 30) auf dem Katheterführungsdraht (18) problemlos unterscheidbar ist, eine Vielzahl von Kathetern mit unterschiedlichen Lagerlängen, wobei jede Katheter-Lagerlänge einem Katheter-Kennzeichen zugeordnet ist, welches eine direkte visuelle Übereinstimmung mit einem einzigartigen Kennzeichen (24, 26, 28, 30) auf dem Führungsdraht (18) hat.

2. Kathetereinführsystem nach Anspruch 1, bei welchem jedes der Vielzahl von einzigartigen Kennzeichen (24, 26, 28, 30) eine Farbe ist.

3. Kathetereinführsystem nach Anspruch 1, bei welchem jedes der Vielzahl von einzigartigen Kennzeichen (24, 26, 28, 30) ein Symbol ist.

4. Kathetereinführsystem nach Anspruch 1, bei welchem jedes der Vielzahl von einzigartigen Kennzeichen (24, 26, 28, 30) ein Code ist, wobei der Code ein oder mehrere Symbole aufweist.

5. Kathetereinführsystem nach Anspruch 1, bei welchem jedes der Vielzahl von einzigartigen Kennzeichen (24, 26, 28, 30) auf eine Basis (22) gedruckt ist.

6. Kathetereinführsystem nach Anspruch 5, bei welchem die Basis (22) eine derartige Farbe hat, dass sie einen Kontrast zwischen einer Farbe des Führungsdrahts (18) und einer Farbe des einzigartigen Kennzeichens (24, 26, 28, 30) bildet.

7. Kathetereinführsystem nach Anspruch 5, bei welchem die Basis (92) weiß ist.

8. Kathetereinführsystem nach Anspruch 1, bei welchem das distale Ende (32) des Führungsdrahts (18) mit einer flexiblen J-Form versehen ist.

9. Kathetereinführsystem nach Anspruch 1, bei welchem der Katheter dem ihm entsprechenden einzigartigen Führungsdraht-Kennzeichen (24, 26, 28, 30) durch ein einzigartiges Katheter-Kennzeichen zugeordnet ist, das direkt auf den Katheter gedruckt ist.

10. Kathetereinführsystem nach Anspruch 1, bei welchem jeder Katheter in einer Katheterverpackung enthalten ist und dem ihm entsprechenden einzigartigen Führungsdraht-Kennzeichen (24, 26, 28, 30) durch ein auf der Katheterverpackung aufgedrucktes einzigartiges Katheter-Kennzeichen zugeordnet ist.

## Revendications

1. Système d'insertion de cathéter comprenant: un fil de guidage de cathéter flexible (18) ayant une extrémité distale (32), une extrémité proximale (20) et une portion centrale d'une longueur prédéterminée présentant une pluralité d'identificateurs uniques (24, 26, 28, 30), chaque identificateur unique (24, 26, 28, 30) étant différenciable facilement de chacun des autres identificateurs uniques (24, 26, 28, 30) sur le fil de guidage de cathéter (18), une pluralité de cathéters de longueurs variables en réserve, chaque longueur de cathéters en réserve étant associée à un identificateur de cathéter qui présente une correspondance visuelle directe avec un identificateur unique (24, 26, 28, 30) sur le fil de guidage (18).

2. Système d'introduction de cathéter selon la revendication 1, dans lequel chacun des plusieurs identificateurs uniques (24, 26, 28, 30) est d'une couleur.

3. Système d'introduction de cathéter selon la revendication 1, dans lequel chacun des plusieurs identificateurs uniques (24, 26, 28, 30) a un symbole.

4. Système d'introduction de cathéter selon la revendication 1, dans lequel chacun des plusieurs identificateurs uniques (24, 26, 28, 30) est un code, le code comprenant un ou plusieurs symboles.

5. Système d'introduction de cathéter selon la revendication 1, dans lequel chacun des plusieurs identificateurs uniques (24, 26, 28, 30) est imprimé sur une base (22).

6. Système d'introduction de cathéter selon la revendication 1, dans lequel la base (22) est d'une couleur de sorte qu'il est prévu un contraste entre la couleur du fil de guidage (18) et une couleur de l'identificateur unique (24, 26, 28, 30).

7. Système d'introduction de cathéter selon la revendication 5, dans lequel la base (22) est blanche.

8. Système d'introduction de cathéter selon la revendication 1, dans lequel l'extrémité distale (32) du fil de guidage (18) est dotée d'une forme en J flexible.

9. Système d'introduction de cathéter selon la revendication 1, dans lequel le cathéter est associé à l'identificateur unique de fil de guidage (24, 26, 28, 30) auquel il correspond par un identificateur unique de cathéter imprimé directement sur le cathéter.

10. Système d'introduction de cathéter selon la revendication 1, dans lequel chaque cathéter est contenu dans un paquet de cathéter et est associé à l'identificateur unique de fil de guidage (24, 26, 28, 30) auquel il correspond par un identificateur unique de cathéter imprimé directement sur le cathéter.
